# EUROPEAN PATENT APPLICATION

(11) **EP 3 505 172 A1**
(43) Date of publication of application: **03.07.2019**
(21) Application number: 17020597.5
(22) Date of filing: 31.12.2017
(51) Int. Cl.: A61K 31/47, A61K 31/495, A61K 31/496, A61P 37/08, A61P 11/06, A61K 9/20

(54) **A PHARMACEUTICAL COMPOSITION COMPRISING MONTELUKAST AND LEVOCETIRIZINE**

(71) Applicant: Abdi Ibrahim Ilac Sanayi ve Ticaret A.S., 34500 Esenyurt/Istanbul (TR)
(72) Inventor: Tolga, Aktas, Esenyurt - Istanbul (TR); Hatice, Canbagi Yildiz, Esenyurt - Istanbul (TR)

(57) **Abstract**

The present invention relates to a solid oral pharmaceutical composition comprising Montelukast, Levocetirizine or pharmaceutically acceptable salt, ester, hydrate, solvate or derivatives thereof, at least one sugar alcohol and at least one pharmaceutically acceptable excipient and the production method thereof.

## Description

### Technical Field

The present invention relates to a solid oral pharmaceutical composition comprising Montelukast, Levocetirizine or pharmaceutically acceptable salt, ester, hydrate, solvate or derivatives thereof, at least one sugar alcohol and at least one pharmaceutically acceptable excipient and the production method thereof.

### Background of the Invention

Montelukast is strong and selective antagonist of leukotriene D4 (LTD4), which acts on the cysteinyl leukotriene receptor (CysLT1). It is used in treatment of asthma and related disorders, and seasonal allergies. Montelukast blocks activity of leukotriene D4, which is on leukotriene receptor CysLT1 in the lung or bronchus tubes connected to the lung, thus, narrowing of bronchus lumen is reduced. Montelukast is on the market with trademark of Singulair® in America and many other countries. In the Indian market, it is sold by the Indian company Cipla with trademark of the Montair®. IUPAC nomenclature of Montelukast is (R,E)-2-(1-((1-(3-(2-(7-Chloroquinolin-2-yl)vinyl)phenyl)-3-(2-(2-hydroxypropan-2-yl)phenyl)propylthio)methyl)cyclopropyl)acetic acid and its chemical structure is shown below.

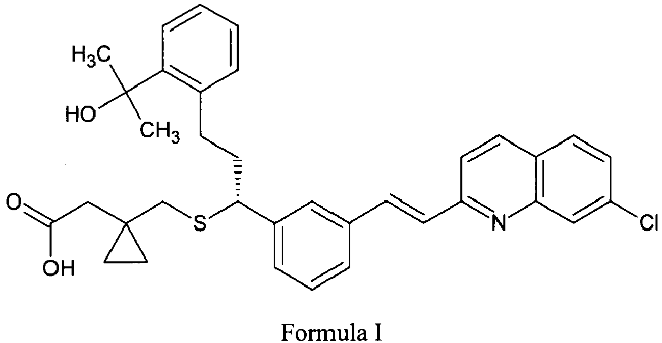

Levocetirizine, the piperazine derivative, is a strong and selective peripheral H1-receptor antagonist. Levocetirizine, the enantiomer of cetirizine (R), inhibits the binding of histamine, which is normally produced by body's immune system and causes symptoms by initiating blood flow to the allergic condition, to HI receptors. For this reason, levocetirizine is used in the treatment of disorders such as hay fever, inflammation in the nose, skin and respiratory tract, lachrymation and itching in the eye, nasal discharge, sneezing and nasal congestion. IUPAC nomenclature of levocetirizine is 2-(2-{4-[(R)-(4-Chlorophenyl)(phenyl)methyl]piperazin-1-yl}ethoxy)acetic acid and its chemical structure is shown below.

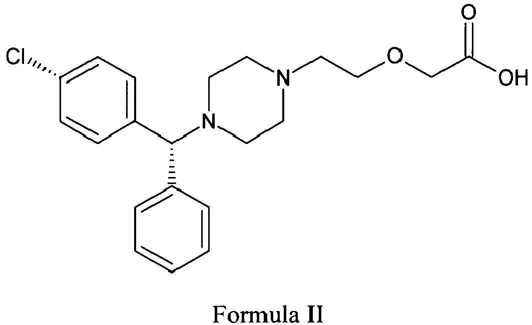

Montelukast having low solubility and high permeability is classified as Class II according to the Biopharmaceutical Classification System (BCS), and levocetirizine having high solubility and low permeability is classified as Class III according to Biopharmaceutical Classification System (BCS).

Compability studies about combination comprising montelukast, levocetirizine and excipients and knowledge in the literature show that the montelukast and levocetirizine are physically and chemically incompatible. This is because montelukast is stable in basic medium whereas levocetirizine is stable in acidic medium. Therefore, contact of montelukast and levocetirizine in monolayer tablets impairs the stability of the composition comprising active ingredients mentioned. (RT Rathod, D Misra; FDA of Montelukast with Levocetirizine: focus on bilayer technology).

The patent application TR2013/02472 proposes filling montelukast sodium granules and levocetirizine tablets together into capsules to eliminate the problems of stability and incompatibility. In this way, it is aimed to prevent the contact of Montelukast with Levocetirizine. The production of fixed dose combination of Montelukast and Levocetrizine by this method is laborious and long-lasting.

The patent application WO2010107404 relates to pharmaceutical compositions comprising Montelukast and Levocetirizine in combination. This document describes that since montelukast is a hygroscopic molecule, the diluent in the composition should not be in hygroscopical structure in order to keep Montelukast's stability and to prevent Montelukast to hold moisture. For this reason, it is stated in this patent application that usage of mannitol as a diluent would be good for Montelukast. However, the mannitol required to maintain the stability of Montelukast causes degradation of Levocetirizine. In this sense, the usage of mannitol is only possible when pharmaceutical composition comprising Montelukast and Levocetirizine is produced in bilayer tablet.

However, there are some difficulties encountered in the production of the bilayer dosage form. These difficulties are that;
- Separation of layers in the tablet as a result of layers not binded each other completely,
- Cross contamination; contamination of granules of one layer to other layer.
- Reduction in efficiency due to the application of dust collection in production to prevent cross contamination and the loss due to this process
- Equipments used in the production are expensive and velocity of compression process of bilayer tablets is slower than those for monolayer tablets.
- Depending on low velocity of compression of bilayer tablet, duration of compression tablet takes longer time and increases energy consumption.

Inventors surprisingly found a stable pharmaceutical composition by formulazing montelukast and levocetirizine together that prevents problems mentioned above.

### The Brief Description of Invention

The present invention relates to pharmaceutical composition and production method of this pharmaceutical composition comprising Montelukast and Levocetrizine meeting needs and eliminating the problems mentioned above.

The main objective of the invention is obtain pharmaceutical composition which solves the problem of incompatibility of montelukast and levosetirizin is suitable for different types of dosage forms without the need for a bilayer tablet.

In the present invention, the pharmaceutical composition comprising Montelukast and Levocetirizine in combination, providing higher proportion of healing and relieving the symptoms in comparison to using said active ingredients separately is provided.

Another objective of the present invention is to obtain a pharmaceutical composition which allows the use of sugar alcohol which can not be used for levosetirizine as a diluent in prior art applications and which is good for montelukast.

Another objective of the invention is to produce stable fixed dose combination comprising Montelukast and Levocetrizine with a cost effective, simple and time-saving production process.

### The Detailed Description of Invention

The present invention relates to a solid oral pharmaceutical composition comprising montelukast, levocetirizine or pharmaceutically acceptable salt, ester, hydrate, solvate or derivatives thereof, at least one sugar alcohol and at least one pharmaceutically acceptable excipient and the production method thereof.

The term "Levosetirizin" used herein refers to Levocetirizine and pharmaceutically acceptable salt, solvate, polymorph, hydrate, enantiomer, derivative or mixtures thereof. Preferably, Levocetirizine dihyrochloride is used in composition.

The term "Montelukast" used herein refers to Montelukast and pharmaceutically acceptable salt, solvate, polymorph, hydrate, enantiomer, derivative or mixtures thereof. Preferably, Montelukast Sodium is used in composition.

In the present invention, the pharmaceutical composition comprises 1 mg to 100 mg Montelukast, preferably 1 mg to 20 mg Montelukast and 1 mg to 100 mg Levocetirizine, preferably 1 mg to 10 mg Levocetirizine.

Solid oral dosage forms may be tablet, capsule, powder, granule, sachet, chewable tablet and is not limited thereto. Preferably, the solid oral dosage form is monolayer tablet.

In the present invention, sugar alcohol is poliol derivative compound comprising hydroxyl group. Sugar alcohol is selected from mannitol, sorbitol, xylitol or mixtures thereof and is not limited thereto. Preferably, it is mannitol.

Filler, masking agent, sweeteners, colorant agent, lubricant, glidant, disintegrant and binder can be used in pharmaceutical composition as pharmaceutically acceptable excipients.

In the present invention, filler refers to calcium carbonate, calcium phosphate, dibasic calcium phosphate, tribasic calcium sulfate, calcium carboxymethyl cellulose, cellulose, dextrin derivatives, dextrin, dextrose, fructose, lactose, lactose derivatives, methylcellulose polymers, hydroxyethylcellulose, hydroxypropylcellulose, L-hydroxypropylcellulose, hydroxypropyl methylcellulose, microcrystalline cellulose, sodium carboxymethyl cellulose, carboxymethylene, carboxymethyl hydroxyethyl cellulose and other cellulose derivatives, starch or modified starch, magnesium carbonate, magnesium oxide, maltodextrin, maltose, sucrose or mixtures thereof and is not limited thereto. Preferably, it is microcrystalline cellulose. Fillers mentioned herein are commonly used in solid dosage forms such as tablet, capsule, MR tablet, orally disintegrating tablet (ODT), chewable tablet and lozenges.

In the present invention, the masking agent is selected from methacrylic acid divinyl benzene, hydroxypropylcellulose derivatives, hydroxypropylmethylcellulose derivatives, polyvinylpyrrolidone derivatives, polyethylene oxide derivatives, polyvinylalcohol derivatives, anion and cation exchange resin or mixtures thereof and is not limited thereto. Preferably it is methacrylic acid divinyl benzene.

In the pharmaceutical composition, the ratio of the weight of the levocetirizine and/or pharmaceutically acceptable salt, ester, hydrate, solvate or derivative thereof to the weight of the masking agent is between 1:0.5 and 1:3, preferably it is 1:2.

In the present invention, sweetener is selected among one of aspartame, acesulfame K, sucrose, sucralose, saccharin, dextrose, sodium cyclamate, cherry flavor, strawberry flavor, orange flavor, mint flavor, lemon flavor, sour-cherry flavor, peach flavor, apricot flavor, vanilla flavor, coffee flavor, raspberry flavor or mixtures thereof and is not limited thereto. Preferably, it is aspartame, acesulfame K ve cherry flavor. Sweetener is added in order to mask bitter taste of the composition and increase patient compliance.

In the present invention, coloring agent is selected from red iron oxide, titanium oxide, iron oxide derivatives, beta carotene, sunset yellow, indigo carmin and/or natural coloring agents or mixtures thereof and is not limited thereto. This colorized agent has a function of protection against light, making opaque, facilitating to distinguish, providing aesthetic appearance. Preferably, red iron oxide is used as colorant agent. Coloring agents may comprise one or more FDA approved colors for oral administration.

Lubricants are commonly used to prevent sticking of powder/granule to machine surface. Especially, in the process of tablet compression, they are used to prevent powder to stick on punch. In the present invention, stearic acid salts such as magnesium stearate, magnesium stearate, palmitic acid salts, oleic acid salts, sodium lauryl sulfate, magnesium lauryl sulfate, hydrogenated vegetable oil, talc, sodium stearil fumarate, macrogol, PEG derivatives or mixtures thereof and is not limited thereto can be used as lubricant. Preferably, it is magnesium stearate.

Particularly in the solid dosage forms, the flowing of powder/granule is regulated by glidants. In the present invention, glidant is selected from colloidal silicon dioxide, silica derivatives, aluminum silicate, magnesium silicate, talc, starch or mixture thereof and is not limited thereto.

In the present invention, disintegrant is selected from crospovidone, sodium starch glycolate, hydroxypropyl starch, microcrystalline cellulose, carboxymethyl cellolose sodium or calcium, croscarmellose sodium, pregelatinized starch, polacrilin potassium, polyplasdone, hydroxypropyl cellulose derivatives, sodium or calcium alginate, agar, guar gum, chitosan, alginic acid or mixtures thereof and is not limited thereto. Preferably, croscarmellose sodium and hydroxypropyl cellulose are used.

In the present invention, binders is selected from polyvinylpyrrolidone (PVP, povidone), polyethylene glycol (PEG), crosslinked polyvinylpyrrolidone, cellulose derivatives (hydroxymethyl cellulose, hydroxypropyl cellulose, carboxy -methylcellulose sodium, ethyl cellulose, hydroxyethyl cellulose, hydroxypropyl methylcellulose), sucrose, alginic acid or sodium alginate, carbomer, cottonseed, dextrin, dextrose, guar gum, hydrogenated vegetable oil type I, magnesium aluminum silicate, maltodextrin maltose, polydextrose, polyethylene oxide and stearic acid or mixtures thereof and is not limited thereto.

In one embodiment of the invention, pharmaceutical composition comprises Montelukast in the range of 0.01 to 20% and Levocetizine in the range of 0,01 to 10 % by weight of the total weight of the pharmaceutical composition.

In another embodiment of the invention, pharmaceutical composition comprises sugar alcohol in the range of 10 to 90%, filler in the range of 10 to 90%, disintegrant in the range of 1 to 10% and lubricant in the range of 0,25 to 5 % by weight of the total weight of the pharmaceutical composition.

In a preferred embodiment of the invention, pharmaceutical composition comprises sugar alcohol in the range of 60 to 65% , filler in the range of 20 to 22% , disintegrant in the range of 1 to 10%, coloring agent in the range of 0.001 to 1%, sweetener in the range of 0.01 to 5% and lubricant in the range of 0,5 to 2% by weight of the total weight of the pharmaceutical composition.

One embodiment of the invention is to obtain stable solid oral pharmaceutical composition comprising Montelukast, Levocetirizine or pharmaceutically acceptable salt, ester, hydrate, solvate, polymorph or derivative thereof, at least one sugar alcohol and at least one pharmaceutically acceptable excipient wherein Montelukast and Levocetirizine is formulized together.

Another embodiment of the invention is to obtain stable solid oral pharmaceutical composition comprising Montelukast, Levocetirizine or pharmaceutically acceptable salt, ester, hydrate, solvate, polymorph or derivative thereof, at least one sugar alcohol, at least one masking agent and at least one pharmaceutically acceptable excipient wherein Montelukast and Levocetirizine is formulized together. Also, the process for production of this pharmaceutical composition comprises steps given below.
a) Dissolving Levocetirizine or pharmaceutically acceptable salt, ester, hydrate, solvate, polymorph or derivative thereof in appropriate solvent to prepare solution,
b) Adding masking agent into the solution obtained in step A and preparing the dispersion,
c) Activating the masking agent by adding pH regulating agent to the dispersion,
d) Drying of dispersion obtained and obtaining Levocetirizine granules, preferably sieveing it,
e) Adding Montelukast or pharmaceutically acceptable salt, ester, hydrate, solvate, polymorph or derivative thereof, sugar alcohol and filler to obtained Levocetrizine granules mixing them and preferably sieving,
f) Adding preferably coloring agent and sweetener to the obtained mixture, mixing and preferably sieveing it,
g) Adding disintegrant to the mixture obtained in (f), preferably sieveing it,
h) Adding lubricant to the mixute, preferably sieving it,
i) Mixture is brought to desired dosage form.

Sugar alcohol can be added to the pharmaceutical composition as two parts. First part, it together with filler can be mixed with Levocetirizine granules. (Step (e) mentioned above). Second part it can be added to the mixture obtained by adding sugar alcohol to the first part. (step (f) mentioned above.)

pH regulating agents used in the production of the pharmaceutical composition of the invention can be selected from sodium hydroxide, sodium bicarbonate, sodium carbonate, potassium hydroxide, trisodium citrate. Preferably, pH regulating agent is sodium hydroxide.

Another embodiment of the invention relates to a stable monolayer tablet composition comprising Montelukast, Levocetirizine or a pharmaceutically acceptable salt, ester, hydrate, solvate or derivative thereof, at least one masking agent, at least one sugar alcohol and at least one pharmaceutically acceptable excipient and a production method thereof.

Another embodiment of the invention relates to stable monolayer tablet composition comprising Montelukast, Levocetirizine or a pharmaceutically acceptable salt, ester, hydrate, solvate or derivative thereof, at least one masking agent, at least one sugar alcohol and at least one pharmaceutically acceptable excipient and a production method comprising the steps of:
a) Dissolving Levocetirizine or pharmaceutically acceptable salt, ester, hydrate, solvate, polymorph or derivative thereof in appropriate solvent to prepare solution,
b) Adding masking agent into the solution obtained in step A and preparing the dispersion,
c) Activating the masking agent by adding pH regulating agent to the dispersion,
d) Drying of dispersion obtained in step c) and getting Levocetirizine granules, preferably sieveing it,
e) Adding Montelukast or pharmaceutically acceptable salt, ester, hydrate, solvate, polymorph or derivative thereof, sugar alcohol to obtained Levocetrizine granules mixing them and preferably sieving,
f) Adding filler and preferably coloring agent and sweetener to the obtained mixture, mixing and preferably sieveing it,
g) Adding disintegrant to the mixture obtained in (f), preferably sieveing it,
h) Adding lubricant to the mixute, preferably sieving it,
i) Compressing the final mixture in the form of monolayer tablet

In the present invention, sieving is carried out by using 1.0 mm sieves or smaller.

### Samples:

The present invention will be described in more details with the following samples, but the scope of the invention is not limited thereto.

**Sample 1:**

| **Ingredients** | **(%)** |
|---|---|
| Montelukast Sodium | 0.01 - 20 |
| Levosetirizin Dihydrochloride | 0.01 - 10 |
| Methacrylic Acid Divinyl Benzene | The ratio of weight of Levocetirizine / Metacrylic acid divinyl benzene is 1:3 |
| Mannitol | 10-90 |
| Microcrystalline Cellulose | 10-90 |
| Red Iron Oxide | 0.01-1 |
| Aspartame | 0.01-5 |
| Acesulfame K | 0.01-5 |
| Cherry Flavor | 0.01-5 |
| Hydroxypropyl Cellulose | 1-10 |
| Croscarmellose Sodium | 1-10 |
| Magnesium Stearate | 0.25 - 5 |
| Pure Water | q.s |
| NaoH | q.s |

**Sample 2:**

| **Ingredients** | **(%)** |
|---|---|
| Montelukast Sodium | 0.01 - 20. |
| Levosetirizin Dihydrochloride | 0.01 - 10 |
| Methacrylic Acid Divinyl Benzene | The ratio of weight of Levocetirizine / Metacrylic acid divinyl benzene is 1:2 |
| Mannitol | 10-90 |
| Microcrystalline Cellulose | 10-90 |
| Red Iron Oxide | 0.01-1 |
| Aspartame | 0.01-5 |
| Acesulfame K | 0.01-5 |
| Cherry Flavor | 0.01-5 |
| Hydroxypropyl Cellulose | 1-10 |
| Croscarmellose Sodium | 1 - 10 |
| Magnesium Stearate | 0.25 - 5 |
| Pure Water | q.s |
| NaoH | q.s |

### Production Process of Sample 1 and Sample 2:

a) Levocetirizine dihydrochloride is dissolved in water,
b) Metacrylic acid divinyl benzene is added into the solution obtained in step A and dispersion is prepared,
c) Masking agent is activated by adding NaOH to the dispersion,
d) Dispersion obtained in step c) is awaited, dried and getting Levocetirizine granules by sieveing it,
e) Montelukast sodium and mannitol is added to obtained Levocetrizine granules, mixed and sieved,
f) Mannitol and red iron oxide is added to the mixture and then sieved,
g) Sweetener is added to the mixture obtained in (f), mixed and then sieved
h) Croscarmellose sodium is added to the mixute and sieved,
i) magnesium stearate is added to the mixture and then sieved,
i) Compressing the final mixture in the form of monolayer tablet Ömek 1 ve Ömek 2'ye ait Ba 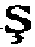 lang 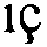 ve 1. Ay 40 °C sonu 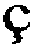 lar 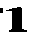 Tablo l'de özetlenmi 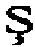 tir. Ge 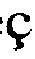 imsizli 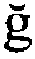 i giderilmi 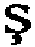 , stabil farmasötik bile 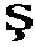 im elde edilmi 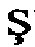 tir.

The initial results and one month stability of the composition (40 °C) are summarized in Table 1. Stable pharmaceutical composition in which incompability problem is solved is obtained.

**Table 1. Total Impurity (Sample 1 and Sample 2)**

| **Total Impurity** | **Limit** | **Initial** | **1^{st} Month 40 °C** |
|---|---|---|---|
| **Sample 1** | Max. % | 0.650 | 2.051 |
| **Sample 2** | 5.0 | 0.431 | 1.247 |

## Claims

1. A solid oral pharmaceutical composition comprising Montelukast, Levocetirizine or pharmaceutically acceptable salt, ester, hydrate, solvate or derivatives thereof, at least one sugar alcohol and at least one pharmaceutically acceptable excipient wherein Montelukast and Levocetrizine is formulized together.

2. A pharmaceutical composition according to Claim 1, it comprises Montelukast sodium.

3. A pharmaceutical composition according to Claim 1, wherein it comprises Levocetirizine dihidrochloride.

4. A pharmaceutical composition according to Claim 1, wherein sugar alcohol is selected from xylitol, mannitol and sorbitol.

5. A pharmaceutical composition according to Claim 1, wherein sugar alcohol is mannitol.

6. A pharmaceutical composition according to Claim 1, wherein it comprises filler, disintegrant, binder, masking agent, coloring agent, sweetener, glidant and lubricant.

7. A pharmaceutical composition according to Claim 1 or Claim 6, wherein filler is selected from calcium carbonate, calcium phosphate, dibasic calcium phosphate, tribasic calcium sulfate, calcium carboxymethyl cellulose, cellulose, dextrin derivatives, dextrin, dextrose, fructose, lactose, lactose derivatives, methylcellulose polymers, hydroxyethylcellulose, hydroxypropylcellulose, L-hydroxypropylcellulose, hydroxypropyl methylcellulose, microcrystalline cellulose, sodium carboxymethyl cellulose, carboxymethylene, carboxymethyl hydroxyethyl cellulose and other cellulose derivatives, starch or modified starch, magnesium carbonate, magnesium oxide, maltodextrin, maltose, sucrose or mixtures thereof.

8. A pharmaceutical composition according to Claim 1 or Claim 6 or Claim 7, wherein filler is microcrystalline cellulose.

9. A pharmaceutical composition according to Claim 1 or Claim 6, wherein masking agent is selected from methacrylic acid divinyl benzene, hydroxypropylcellulose derivatives, hydroxypropylmethylcellulose derivatives, polyvinylpyrrolidone derivatives, polyethylene oxide derivatives, polyvinylalcohol derivatives, anion and cation exchange resin or mixtures thereof.

10. A pharmaceutical composition according to Claim 1 or Claim 6 or Claim 9, wherein masking agent is methacrylic acid divinyl benzene.

11. A pharmaceutical composition according to Claim 1 or Claim 6, wherein lubricant is selected from stearic acid salts such as magnesium stearate, magnesium stearate, palmitic acid salts, oleic acid salts, sodium lauryl sulfate, magnesium lauryl sulfate, hydrogenated vegetable oil, talc, sodium stearil fumarate, macrogol, PEG derivatives or mixtures thereof.

12. A pharmaceutical composition according to Claim 1 or Claim 6 or Claim 11, wherein lubricant is magnesium stearate.

13. A pharmaceutical composition according to Claim 1 or Claim 6, wherein disintegrant is selected from crospovidone, sodium starch glycolate, hydroxypropyl starch, microcrystalline cellulose, carboxymethyl cellolose sodium or calcium, croscarmellose sodium, pregelatinized starch, polacrilin potassium, polyplasdone, hydroxypropyl cellulose derivatives, sodium or calcium alginate, agar, guar gum, chitosan, alginic acid or mixtures thereof.

14. A pharmaceutical composition according to Claim 1 or Claim 6 or Claim 13, wherein disintegrant is croscarmellose sodium and hydroxypropyl cellulose.

15. A pharmaceutical composition according to preceeding claims, the ratio of weight of Levocetirizine and / or pharmaceutically acceptable salt, ester, hydrate, solvate or derivatives thereof to the weight of masking agent is between 1:0.5 and. 1:3.
